# EUROPEAN PATENT SPECIFICATION

(11) **EP 0 627 081 B1**
(45) Date of publication and mention of the grant of the patent: **12.03.1997**
(21) Application number: 94903770.9
(22) Date of filing: 14.12.1993
(51) Int. Cl.: G01N 33/543, G01N 33/564, G01N 33/563

(54) **ASSAY METHOD FOR THE DETERMINATION OF AUTOANTIBODIES IN BIOLOGICAL FLUIDS**
VERFAHREN ZUR BESTIMMUNG VON AUTOANTIKÖRPERN IN BIOLOGISCHEN FLÜSSIGKEITEN
PROCEDE DE DETERMINATION D'AUTOANTICORPS DANS DES LIQUIDES BIOLOGIQUES

(30) Priority: 21.12.1992 DE 4243375
(43) Date of publication of application: 07.12.1994
(73) Proprietor: B.R.A.H.M.S Diagnostica GmbH, D-12099 Berlin (DE)
(72) Inventor: BERGMANN, Andreas, D-12351 Berlin (DE); MIKLUS, Metod, D-10965 Berlin (DE); THOMAS, Barbara, D-13357 Berlin (DE); STRUCK, Joachim, D-12247 Berlin (DE)
(74) Representative: Andrae, Steffen, Dr.
(86) International application number: EP9303528
(87) International publication number: WO9415214

(56) References cited:
- EP-A- 0 442 231
- WO-A-93/00587
- US-A- 4 292 403
- CLINICAL CHEMISTRY vol. 35, no. 9 , 1989 pages 1949 - 1954 K.BEEVER ET AL. 'Highly Sensitive Assays of Autoantibodies to Thyroglobulin and to Thyroid Peroxidase.' cited in the application
- CLINICAL CHEMISTRY, vol. 35, no. 9, 1989; K. BEEVER et al., pp. 1949-1954/

## Description

The invention relates to an immunological assay method for the determination of autoantibodies, the detection of which permits the diagnosis of an autoimmune disease.

Immunological assay methods in many variants play a very important role in medical diagnostics. In addition to those assay methods which aim at the qualitative and/or quantitative determination of antigens or haptens, for example hormones, there are also many such assay methods for the determination of antibodies in biological fluids, in particular human sera.

Antibodies are proteins which are referred to as immunoglobulins (Ig) and which are formed by the body as a reaction to an antigen. Since antigens normally have a large number of antigenic determinants, antibodies are of a polyclonal nature and thus represent a population of proteins having different binding properties with respect to the antigen against which they are directed. They are normally formed against exogenous antigens in order to protect the body from substances which have corresponding antigenic determinants. However, if the immune system of the body incorrectly recognises certain endogenous cells or cell structures as exogenous, it is also possible for antibodies to be formed against antigen determinants of endogenous elements. Such endogenous elements are then referred to as autoantigens, and the antibodies formed against them are referred to as autoantibodies.

Known assay methods for antibodies realise various basic principles in one form or another, two of which are shown schematically, for example, at the top of column 3 in U.S. Patent B1 3 654 090. In a variant which corresponds to the classical radioimmunoassay (RIA), less than the required amount of an immobilised antigen is used, and a labelled form of the antibody to be determined is added in a known amount to the sample to be investigated. From the degree of binding of the labelled antibody to the immobilised antigen, it is possible to obtain information about the presence or concentration of the relevant antibody. For this test operating according to the competition principle, the antigen is required in very pure form.

In a procedure based on a second principle, a known amount of the antibody to be determined or of a suitable derivative thereof is immobilised on a solid substrate, and the antibody present in the sample to be investigated and the immobilised antibody are then allowed to compete for a labelled antigen added to the reaction system. The presence of amount of the antibody to be determined is indicated by the reduction in the binding of the labelled antigen to the immobilised antibody and hence to the solid phase.

In the last-mentioned method of determination, a labelled form of the associated, highly pure antigen is required, and the antibody to be determined and the immobilised antibody must be present in amounts such that effective competition can occur between the immobilised antibody and the antibody in the sample to be investigated, if necessary taking into account the fact that the affinities to the labelled antigen may not be completely identical.

According to a further principle, in a procedure analogous to the sandwich test well known for antigen determination and used for the determination of antibodies, an excess of an antigen, usually in immobilised form, is initially taken and binds the total amount of the antibody to be determined, and, by means of a subsquent second immunological reaction with a second labelled binder against the antibody bound in the first step, the latter is labelled with formation of a sandwich-like immune complex. The second binder is frequently an anti-antibody (double antibody method), or labelling is carried out using labelled unspecific binders, such as the so-called proteins A or G, which are proteins which are obtained from bacteria and bind unspecifically to many IgG antibodies. The selectivity of the method in this variant is ensured by the selectivity of the binding between the immobilised antigen and the antibody against this antigen, which antibody is to be determined, whereas the labelling reaction is rather unspecific.

Another variant of the antibody determination according to the sandwich principle, which is used in particular for the determination of autoantibodies and which constitutes the most closely related prior art which is used as a basis in the present application, differs from the variant just described in that a fairly unspecific binder for antibodies is used for the quantitative binding (extraction from the sample solution) of the antibodies to be determined instead of a selective antigen, and that the selectivity of the assay method is ensured by virtue of the fact that the label used for the "correct" bound antibody is a highly pure labelled antigen against the latter. As in the "competitive" process variant described further above, it has been possible to date to realise this principle only when the antigen in question was available in pure form.

In US-A-4,292,403 a method for the detection and/or determination of immunoglobulins (antibodies) IgX, in particular immunoglobulins of the classes IgM, IgA, IgD and IgE, is disclosed, in which a test medium is reacted with
a) an insolubilized antibody or fragment of an antibody against antibodies of the class IgX to which the antibody to be determined belongs,
b) an antigen for which the antibody to be determined has specific affinity, in the form of a microbial or virus preparation, and
c) labelled fragments of antibodies which have a specific affinity for the antigen in question, especially enzyme-labelled F(ab')₂ fragments of IgG,
and in which in the last step the solid or undissolved phase is separated from the liquid phase, and the presence or amount of the antibodies to be detected in the test medium are determined by measuring the amount of the labelled IgG fragment of bound to the solid phase.

The IgG-fragments in all examples are obtained from sera of immunized animals and positive patients respectively, and, therefore, are polyclonal. The method is a multi-step method comprising several steps and requires a great number of washing steps.

In the determination of antibodies against exogenous antigens, the required functions for the various tests can frequently be fulfilled without considerable difficulties. The antibody concentrations against exogenous antigens are usually relatively low, and the associated antigens or haptens can frequently be synthesised in sufficient amounts by chemical or biotechnological methods or isolated from natural material and concentrated.

However, if it is intended to determine autoantibodies according to one of the principles described, a number of difficulties, some of them considerable, are encountered, both owing to the resulting autoantibody concentrations and owing to the nature of the autoantigens.

The determination of autoantibodies is very important for the detection of the presence of an autoimmune disease, in particular for correctly interpreting the observed disease symptoms and avoiding incorrect treatments which may be harmful. Known autoimmune diseases, some of which are extremely severe, are, for example, rheumatoid arthritis, diabetes mellitus type 1, myasthenia gravis and certain autoimmune diseases associated with the thyroid, such as Graves' disease, myxedemanaemia and Hashimoto's thryoiditis. In the case of the thyroid autoimmune diseases, depending on the type of disease, thryeoglobulin, TSH receptor and/or thryoid peroxidase (TPO) act as autoantigens, recent knowledge indicating that the latter is identical to the so-called microsomal antigen. The present invention is described below in particular with reference to the determination of thryoid autoantibodies, in particular of antibodies against hTPO. However, the novel principle on which the invention is based is not restricted to these specific determinations but can be advantageously used also for the determination of other autoantibodies.

Summaries of the current state of knowledge in the area of thyroid autoimmune diseases are to be found in the scientific literature, for example in the article by Marian Ludgate and Gilbert Vassart in: Autoimmunity, 1990, Volume 7, pages 210-211, and in the review by Jadwiga Furmaniak and Bernard Rees Smith in: Autoimmunity, 1990, volume 7, pages 63-80, and in the article by P.-M. Schumm-Drager and H.J.C. Wenisch in: Akt. Endokr. Stoffw. 10 (1989), pages 9-102 (special issue), which gives a review of the methods for the detection of thyroid autoantibodies.

The immunodiagnostic determination of thyroid autoantibodies or autoantibodies generally using one of the assay types stated at the outset faces the fundamental difficulty that the autoantibodies are very frequently directed against autoantigens which are anchored in the cell membrane and are difficult to obtain in the high purity and amount required for the conventional procedure. Human thyroid peroxidase (hTPO), an enzyme which is the autoantigen responsible for Hashimoto's thyroiditis, is for example a glycosylated haemoprotein which is bound to the thyroid membranes. Its antigenic properties, including the types of epitopes present on its surface, are described in the article by P. Carayon et al. in: Endocrinology, Vol. 125, No. 3, pages 1211 to 1218. In order to have this thyroid peroxidase available as an antigen in sufficient purity and amount for the immunodiagnostic assay method based on the known principle, the thyroid peroxidase must be detached from the membrane by a proteolytic method or with the aid of detergents and purified by means of immunoadsorbents or by means of conventional chromatography methods on various separation phases, for example by gel filtration, ion exchange chromatography, chromatography via hydrophobic interactions, chromatography via aromatic interactions, adsorption chromatography and chromatography using concanavalin A. These methods are expensive and are associated with the risk of unintentional changes in the enzyme to be isolated and a considerable loss of material. Highly purified natural thyroid peroxidase (TPO) is therefore available only in small amounts and at high prices. This fact directly affects all of the above process variants in which the antigen has to be used in pure form either for the selective binding of the antibodies to be determined or as selective labels. As an alternative to the isolation of the thyroid peroxidase from thyroids, experiments have therefore been carried out, and methods developed, in which TPO can be produced by a genetic engineering procedure. However, the TPO obtained in this manner is also available only in limited amounts and at high prices, and it is not certain that the material obtained by genetic engineering is identical in every case with the natural thyroid peroxidase, in particular with regard to the antigenic properties.

A further difficulty arises from the fact that the antigenic properties of the TPO may be very greatly impaired by chemical effects, in particular if the three-dimensional structure is changed and/or the disulphide bridges are broken as a result (cf. the stated article by P. Carayon). However, in order to be able to use TPO as a labelled antigen in the classical method for antibody determination, a label must be chemically bonded to the TPO. In addition to the difficulty of obtaining pure TPO, there is at this stage the risk that the antigenic properties of the TPO would be influenced by the reactions associated with the labelling in such a way that the TPO no longer corresponds to the natural TPO and furthermore is therefore only of limited suitability as an antigen for the detection of autoantibodies. For example, the changes caused by the isolation and/or labelling of the TPO may result in only a part of the polyclonal TPO autoantibodies reacting with such a labelled TPO.

In order to avoid at least some of the problems associated with the isolation and labelling of TPO, a test in which a TPO which is not highly purified but used in crude form was also developed as a modification of the sandwich test, described at the outset, for the determination of antibodies. In this test, however, there is the danger that other substances with antigenic properties may also be present in the immobilised crude TPO and lead to immobilisation of antibodies other than the required ones, and that these are then labelled in the subsequent, relatively unspecific labelling according to a double antibody method or by labelled protein A and give false positive results.

In view of the difficulties described with regard to a determination of autoantibodies by the known immunodiagnostic assay methods, there is therefore an urgent need for an improved method for the immunodiagnostic determination of autoantibodies in biological fluids which permits a safe qualitative determination of antibodies in biological fluids, which is also suitable for the reliable quantitative determination of antibodies as a result of suitable calibration and optimisation of the parameters of the method and in which it is not necessary to use significant amounts of highly purified antigens for the antibodies to be determined. The Applicant's method described in the previously unpublished patent application DE 41 20 412.3 or in the corresponding PCT Application PCT/EP92/01348 achieves these and other objects for the case of antibody assays, in particular autoantibody assays, in which the presence of the relevant autoantibodies manifests itself as a disturbance of the binding of a labelled antibody to the solid phase and in which the autoantigen, in particular TPO, is not required in highly purified form but can be used in the form of a crude, natural organ extract.

Since, in the variant which operates according to the sandwich method and in which the antibodies to be determined, in particular autoantibodies, are extracted by relatively unspecific binding with a "binder", the selectivity of the method depends on the selectivity of the label for the relevant antibodies, the use of a highly purified labelled antigen and hence the acceptance of all the disadvantages described have appeared unavoidable so far in the case of this variant.

It is the object of the present invention to develop a method for the determination of autoantibodies, in which the latter are extracted from the reaction solution by binding to an unspecific binder and are then specifically labelled, and in which the problems associated with the use of highly purified labelled antigens can be solved.

This object is achieved by an immunological assay method as described in Claim 1.

Preferred embodiments are contained in the subclaims.

According to the invention, autoantibodies, such as human autoantibodies against thyroid peroxidase (hTPO), are determined by a procedure in which the antibodies to be determined are bound with the use of unspecific binders in a conventional manner to a solid or undissolved phase and then selectively labelled, for a subsequent measurement, by reaction with a crude antigen preparation and a further, labelled antibody or antibody fragment which are specific for the antigen but are not bound, or not significantly bound, by the binder. The presence of the antibodies to be determined manifests itself in the form of a label bound to the solid or undissolved phase, the amount of said label being proportional to the amount of the antibodies to be determined in the sample.

In the method according to the invention, no purified antigen, for example hPTO, is required, and this antigen furthermore need not be directly labelled. The selectivity of the method is ensured by virtue of the fact that the "correct" sandwiches for the determination are selected from immune complexes or sandwiches which are formed by a sequence of relatively unspecific reactions and consist of binder/(bound antibodies which also comprise the antibodies to be determined) and optionally (crude antigen and accompanying substances) by labelling with a labelled antibody or antibody fragment specific only for the antigen. Neither in the extraction of the antibodies by means of the binder nor in the reaction of the resulting immune complex with the crude antigen can it be assumed that the "correct" sandwiches comprising binder/antibody to be determined/antigen will be formed with a selectivity sufficient for a measurement. However, by carrying out the labelling in such a way that only bound antigens will be selectively detected for the subsequent determination, a generally very specific assay method is obtained.

For practical reasons, or more precisely to reduce the number of reagents supplied to the end user, it is preferable if the reaction between crude antigen preparation and the tracer specific for the antigen is carried out separately, so that the determination is effected using a crude antigen preparation in which only the actual antigens carry a label, but the method can in principle also readily be carried out in such a way that the reaction between the binder, sample fluid and crude antigen preparation is carried out first and labelling is effected subsequently in a separate step, or that all reagents mentioned are incubated simultaneously.

In the present description, the term "antigen" is used in a narrow sense, namely only for those substances which do not simultaneously represent antibodies and have immunoglobulin character, although it is well known that antibodies can also act as antigens and can result in the formation of anti-antibodies. For the purposes of the present description, antigens are in particular those endogenous substances which, as described at the outset, also act as autoantigens and can result in the formation of autoantibodies.

To avoid a cross-reaction with the binder used for extracting the antibodies to be determined, the tracers used are preferably labelled fragments of monoclonal antibodies F(Ak)*, for example fragments of the type designated by F(ab')₂ or F(ab), or other fragments which are directed towards a region of the antigen which is not recognized by autoantibodies. Antibody fragments without the Fc part are preferred. The reason for the preferential use of such antibody fragments is that the unspecific binders, such as protein A or protein G, recognize and bind antibodies via this Fc fragment which is essentially identical for different antibodies. If it is ensured that the antibodies against the antigen which are used for selective labelling do not have this Fc fragment, falsification of the results of the measurement due to direct binding of the tracer to the binder can be prevented.

The invention is illustrated in more detail below with reference to the preferred embodiment.

The Figures show the following:

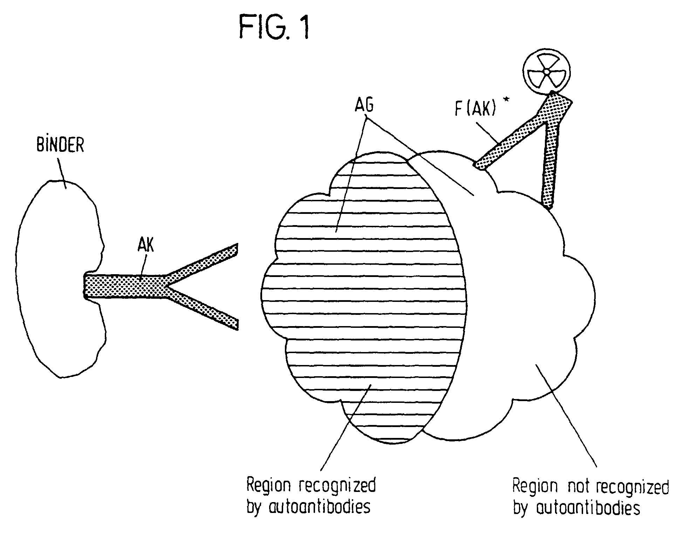

Figure 1 shows a schematic representation of the method according to the invention, the basic components of the method being denoted, for easier identification, by the symbols also used in the Claims, without there being any intention to restrict the particular assay components in any material way by these symbols as such.

Figure 2 shows a typical standard curve of the method described in the Example.

Figure 3 shows a graph with a comparison of the method according to the invention and the basic method commercially available as the Applicant's IMMUtest anti-TPO.

Figure 4 shows a graph with a comparison of the method according to the invention and the assay method commercially available as the Applicant's DYNotest^{R} anti-TPO.

### Example

The following Example shows the procedure and the practical advantages of an assay in which the method according to the invention is realised, with reference to a preferred embodiment which relates to the detection of human autoantibodies against human thyroid peroxidase (hTPO).

It is a further development, according to the invention, of the basic method for the determination of autoantibodies against hTPO, which method is described in principle in the article by Beever et al., Clinical Chemistry 35, 1989, 1949-1954, and realised in practice in the Applicant's commercially available IMMUtest anti-TPO. In this basic method, the autoantibody-containing sample, insoluble protein A for binding the latter (preferably as direct Staphylococcus aureus suspension, as sold by the company Calbiochem under the name Pansorbin^{R}) and directly labelled purified TPO as tracer are brought into contact with one another. Protein A is present in a sufficient excess to bind all antibodies present in the sample. In the presence of autoantibodies against hPTO, a sandwich is formed and the tracer is therefore bound to the undissolved phase. Bound TPO tracer is then separated from dissolved TPO by centrifuging, and the sediment is measured. Purified, directly labelled TPO has been required to date in this basic method.

### 1. Preparation of an hTPO extract from human thyroid

Frozen human thyroids (60 g) were comminuted, a buffer (200 ml of phosphate-buffered salt solution, PBS) were added and homogenisation was then carried out by means of a homogeniser (Ultraturrax from the company IKA Werke). After centrifuging for one hour at 100,000 g, the supernatant solution was removed and the pellet obtained was rehomogenised in the same manner as the comminuted thyroids. This was followed by further centrifuging at 100,000 g for 1 hour. The pellet now obtained was rehomogenised in PBC (200 ml) which additionally contained 0.5% of Triton X 100 from the company PIERCE (Catalogue No. 28314) as a detergent, and stirring was carried out for 1 hour at 4°C. Finally, the homogenate obtained was centrifuged at 100,000 g for 2 hours. The resulting supernatant solution is the hTPO extract which is used as crude natural antigen (Ag) in the method according to the invention for the determination of autoantibodies against hTPO.

### 2. Preparation of a ¹²⁵I-labelled F(ab')₂ fragment of a monoclonal anti-hTPO antibody (F(ab)₂*)

A monoclonal antibody (MAB) which binds to hTPO but whose binding site does not correspond to the region recognized by human anti-hTPO antibodies was chosen. This antibody was prepared and purified according to the publication by J. Ruf et al. (Endocrinology, 1989, 125, pages 1211-1218).

To avoid binding of this antibody to the protein A used as a binder for the autoantibodies to be determined, the Fc moiety was removed from the MAB. For this purpose, the purified MAB (1 mg in 1 ml of 20 mM phosphate buffer) was cleaved with immobilised pepsin (Sigma, catalogue number P 3286) into the Fc and F(ab')₂ fragments in a manner known per se. Incubation was carried out using 1000 units of pepsin per 1 mg of antibodies, for 20 h at 24°C. The fragments obtained were separated by means of gel chromatography (using the Pharmacia system) over a Superose-12 column in an FPLC unit. The purified F(ab')₂ fragments were then labelled with ¹²⁵I by the chloramine T method. For this purpose, 100 µg of the F(ab')₂ fragment in 280 µl of 20 mM phosphate buffer were mixed with 67 MBq Na¹²⁵I (Amersham, No. IMS.30) and 10 µg of chloramine T (Merck, Catalogue No. 2426) for one minute. The labelling mixture was then purified by HPLC to separate free ¹²⁵I from the ¹²⁵I bound to F(ab)₂ fragments. The separation was carried out in an HPLC unit with a Zorbax GF250/9 column.

### 3. Preparation of a tracer complex for the determination of anti-hTPO autoantibodies

For the determination of human autoantibodes against hTPO, in the present case a tracer complex consisting of antigen (Ag from the crude antigen preparation obtained as hTPO extract under 1.) and labelled F(ab')₂ fragments was first prepared. For this purpose, both proteins were mixed in a molar ratio of 1:1 and incubated for 20 h at 24°C. Within this period, the F(ab')₂* binds to the Ag, and the resulting complex is adjusted to an activity (¹²⁵I) and then used in the method according to the invention. The incubation and dilution buffer had the following composition: 20 mM Tris, 150 mM NaCl, 0.1% Tween®, 0.5% BSA (bovine serum albumin) and 3 mM NaN₃.

### 4. Description of the test method for the determination of human anti-hTPO autoantibodies

The following procedure was adopted for the detection of human autoantibodies against hTPO and for the calibration:
1. In each case 50 µl of the sample to be investigated or of the standard or serum were pipetted into test tubes (4.5 ml tapered tubes).
2. 50 µl of a protein A suspension (Calbiochem: Catalogue No. 507858) were then pipetted.
3. Finally, 50 µl of the tracer complex described in point 3. were pipetted, the said complex containing the antigen hTPO in the form of the extract from human thyroids according to point 1. and the labelled F(ab')₂* fragments of the monoclonal anti-hTPO antibody, which fragments are bound to said antigen.
4. The reaction mixture was briefly shaken and then incubated for one hour at room temperature.
5. After the incubation, 1 ml of buffer (20 mM Tris, 150 mM NaCl, 0.05% Tween® detergent and 3 mM NaN₃) was pipetted into each tube.
6. The tubes were then centrifuged for 15 min at 2000 g and decanted, and the remaining sediment was measured in a gamma counter.

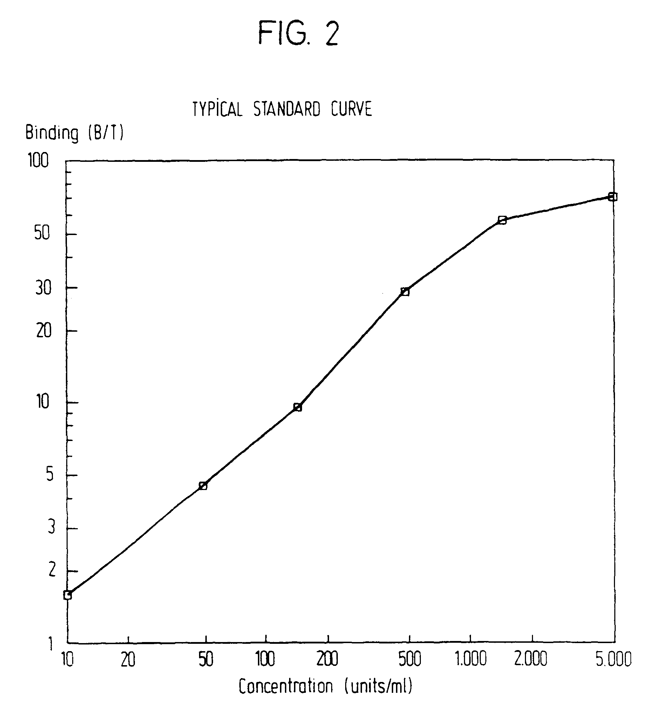

Fig. 2 shows a typical standard curve of this method.

### 5. Clinical data

In a clinical study, the results obtained by the novel method described were compared with those obtained using existing immunological assay methods for the determination of autoantibodies against hTPO for 200 sera.

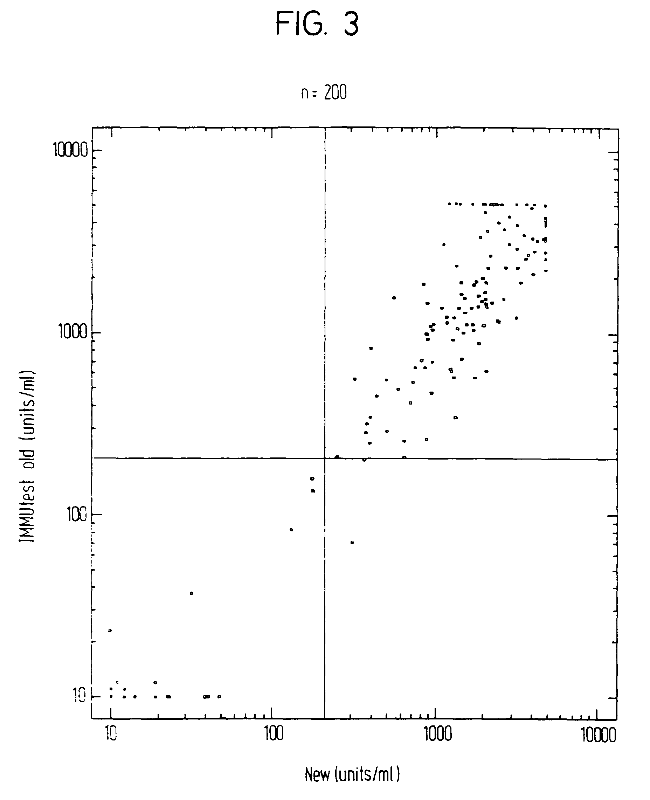

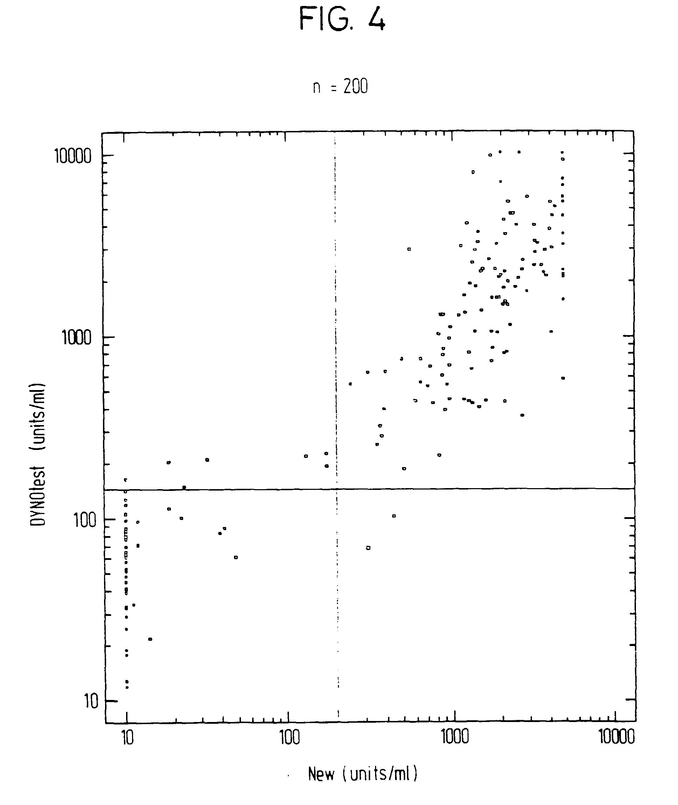

The results are summarised in Table 1 and in Figures 3 and 4.

In Table 1, columns a) and b) relate to the results of assay methods of the prior art, which will be explained in more detail below. On the other hand, the values obtained using the novel method carried out as described above are shown under c). The results of the measurements are stated in units/ml in the Table.

The methods of the prior art which are compared, as comparative methods, with the method according to the invention were specifically as follows:
a) A method in which an anti-hTPO antibody is used on the solid phase. Radiolabelled and purified hTPO is displaced from the solid phase by human autoantibodies in a competitive reaction. The test used is a commercially available test which is on the market as the Applicant's DYNOtest^{R} anti-TPO.
b) In this test according to the above basic method, protein A is immobilised on the solid phase analogously to the method according to the invention. Autoantibodies in a sample are detected by means of their binding to the solid phase and their subsequent detection with the aid of labelled purified hTPO. In the specific case, ¹²⁵I-labelled hTPO is used in accordance with the Applicant's IMMUtest anti-TPO (cf. also Beever et al., Clin. Chem., 1989, 35:1949-1954).

In the evaluation of results of measurements for autoimmune diseases, it has proved useful to define a transition zone, the so-called "grey region", between the clearly negative and clearly positive region. This region is between 100 and 200 units/ml in the method according to the invention and in the comparative method b), and between 70 and 130 units/ml in the method a). When the grey regions are taken into account, the results of the two comparative methods correlate very well with those of method c) according to the invention. If method c) according to the invention is compared with method b), only one out of 200 patient samples is found to exhibit discrepancies, namely to be slightly positive in the method according to the invention and negative in b). In the correlation of methods a) and c), four out of 200 samples are found to be slightly positive in a) whereas they are clearly to be regarded as negative in c). On the other hand, one sample is recognized as slightly positive in c) and negative with the aid of a).

As already mentioned at the outset, the novel principle is not restricted to the determination of autoantibodies against hTPO. Similar advantages are also likely for the determination of other autoantibodies which are formed against membrane-associated and other autoantigens. The determination of autoantibodies against acetylcholine receptors of the nicotine type, the occurrence of which is generally considered to be typical for the severe autoimmune disease myasthenia gravis, may be mentioned in this context.

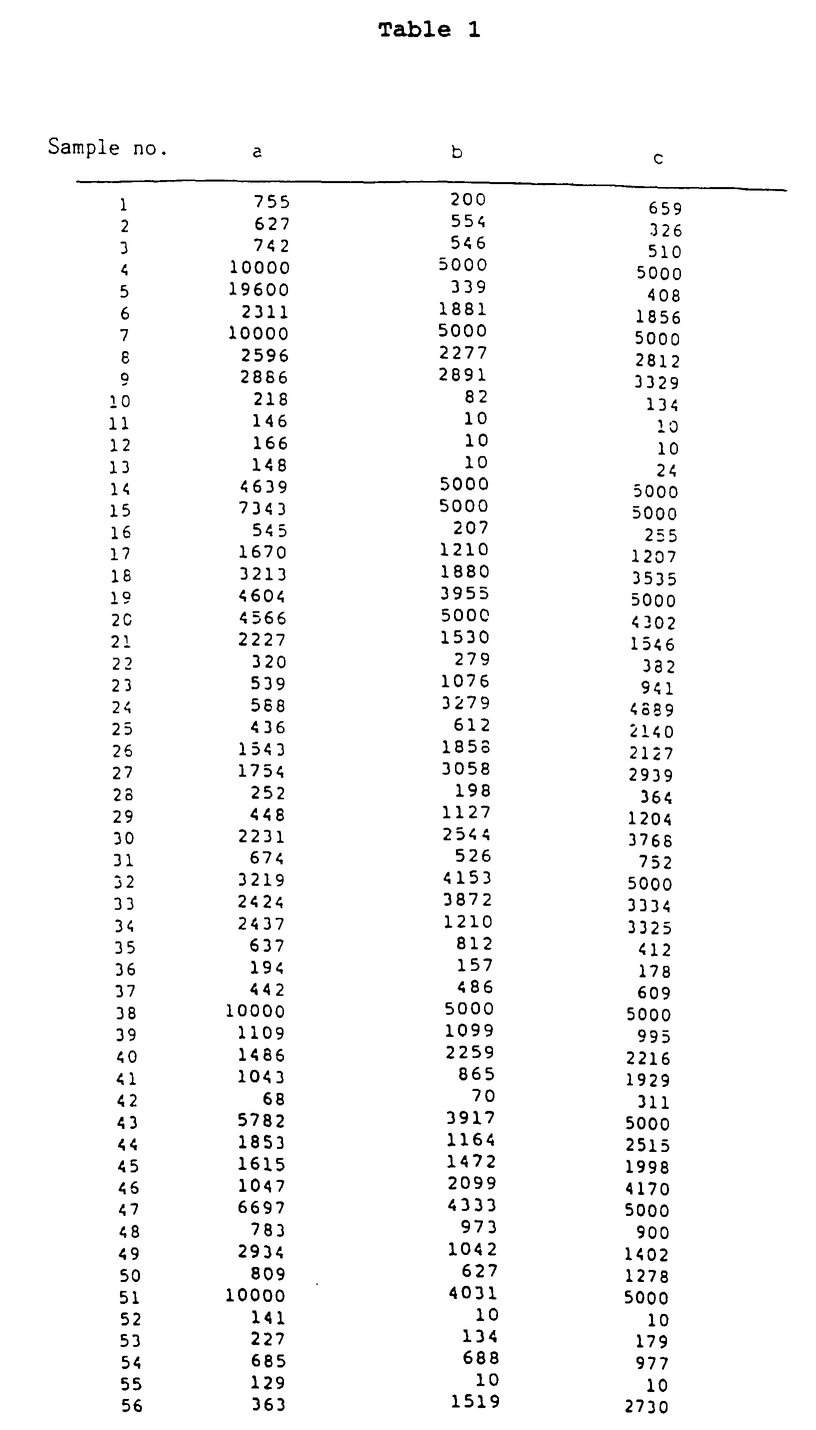

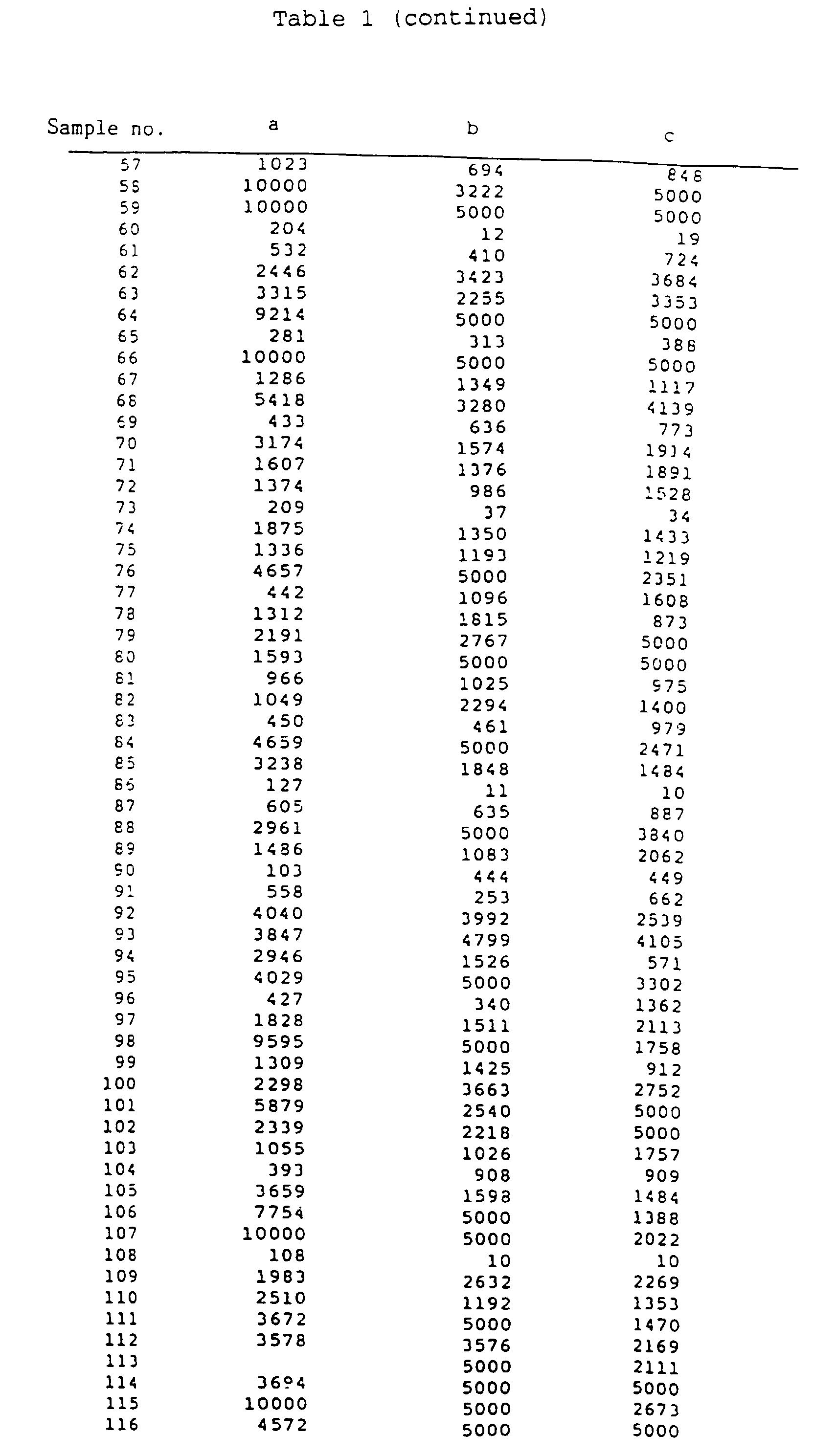

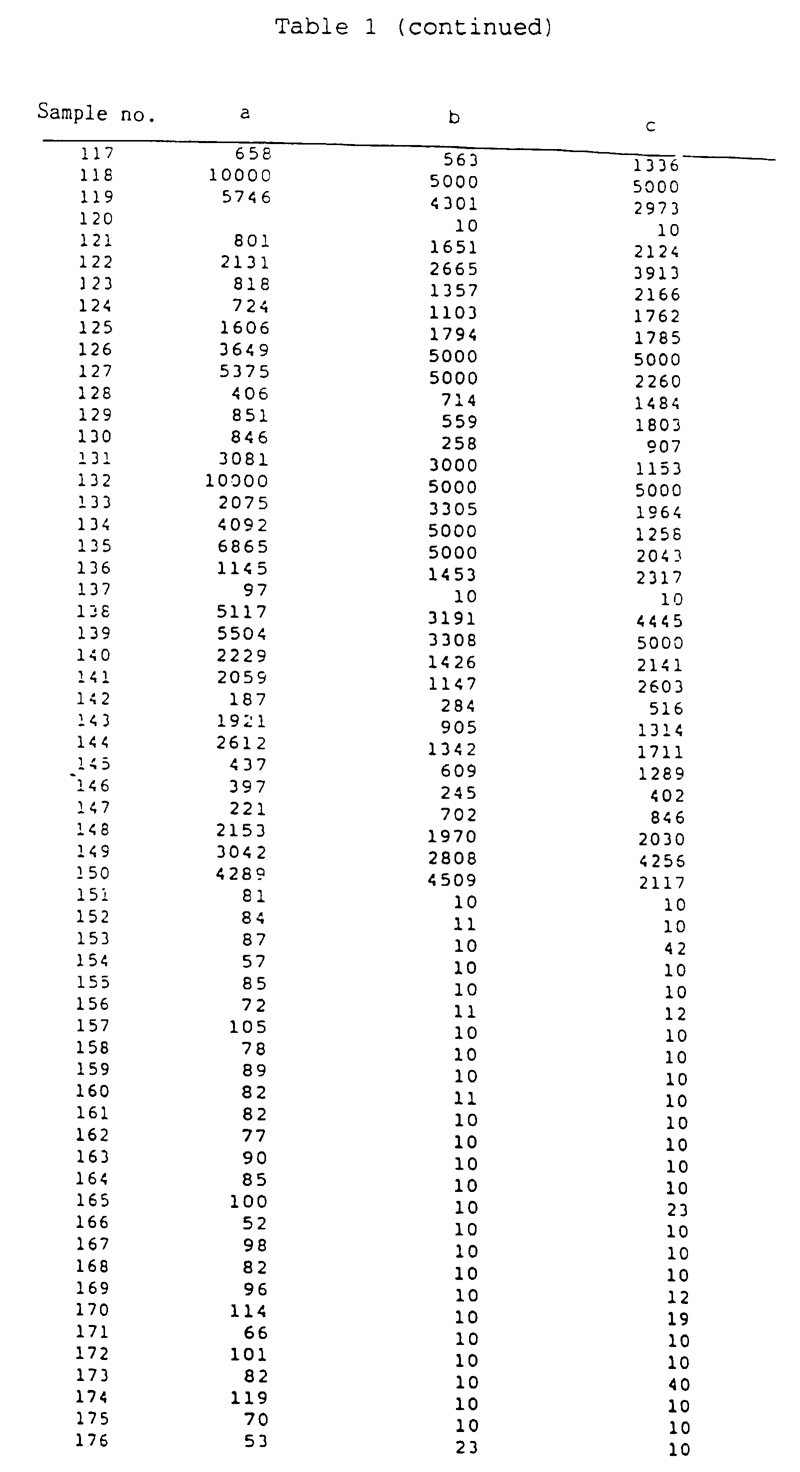

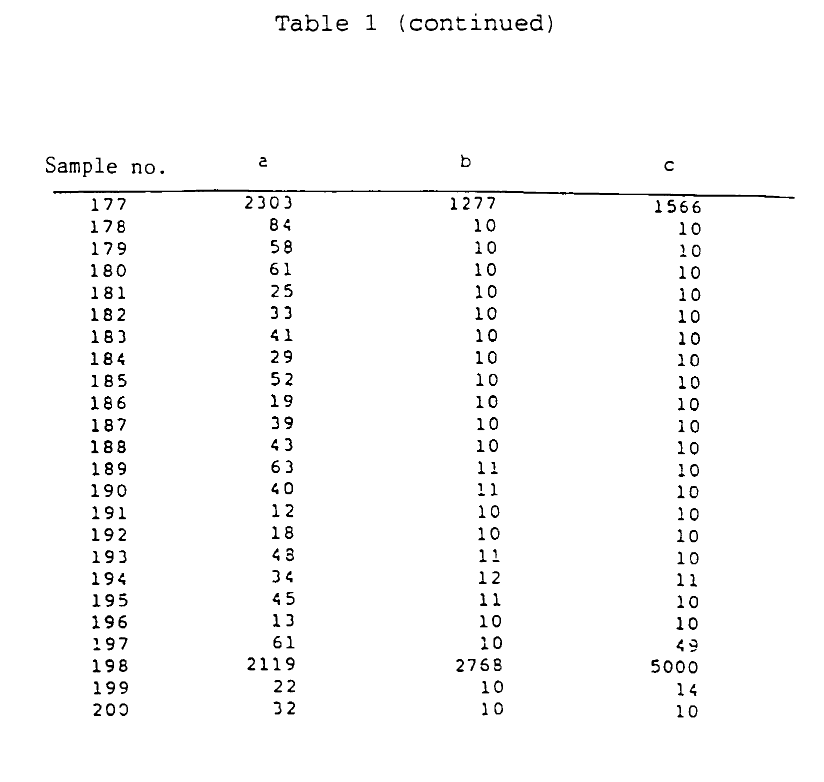

## Claims

1. Immunological assay method for the determination of autoantibodies (Ak) in biological fluids, in which the biological fluid is reacted with
a) an unspecific binder for antibodies of the type of the antibodies to be determined, said binder being insoluble in the reaction mixture or bound to a solid phase or microsolid phase,
b) an antigen (Ag) for the antibodies (Ak) to be determined, and
c) a selective labelling reagent in the form of an antibody or a fragment of an antibody (F(Ak)*) having a detectable label and binding specifically to the antigen (Ag),
and in which the solid or undissolved phase is subsequently separated from the liquid phase and the presence or amount of the antibody (Ak) to be determined in the biological fluid is detected by means of the amount of labelled antibody or labelled fragment of the antibody (F(Ak)*) indirectly bound to the solid or undissolved phase via the unspecific binder, and the presence or the amount of the antibodies to be determined in the biological sample is then determined by qualitative estimation or by computational evaluation of the results obtained, using calibration curves, characterized in that,
in order to determine human autoantibodies (Ak) the detection of which permits the diagnosis of an autoimmune disease, the antigen (Ag) is used as a crude autoantigen preparation, and wherein, as selective labelling reagent, a labelled monoclonal antibody or a labelled fragment of a monoclonal antibody (F(Ak)*) are used, which are not bound, or not significantly bound, by the unspecific binder and which bind to such region of the antigen (Ag), which is not recognized by the autoantibody (Ak) to be determined and which is not affected by the binding of the antigen (Ag) to the autoantibody (Ak) to be determined.

2. Assay method according to claim 1, wherein a human or animal organ extract is used as crude native autoantigen preparation.

3. Assay method according to Claim 1, wherein the unspecific binder is selected from protein A, protein G and anti-human antibodies of non-human origin.

4. Assay method according to Claim 1, 2 or 3, wherein the autoantibodies to be determined are autoantibodies against thyroid peroxidase (TPO) and wherein as crude antigen preparation (Ag) crude natural hTPO in the form of an extract of comminuted human thyroids is used.

5. Assay method according to any of Claims 1 to 4, wherein the labelled monoclonal antibody or the labelled fragment of a monoclonal antibody (F(Ak)*) bear a detectable label commonly used in immunological assay methods, and e.g. selected from a radioactive isotope, an enzyme, a fluorescent or chemiluminescent label or a substrate for an enzymatic detection reaction.

6. Assay method according to any of Claims 1 to 5, wherein the crude native antigen preparation (Ag) is reacted in a preceding stage with the labelled monoclonal antibody or the labelled fragment of a monoclonal antibody (F(Ak)*) and the amount of the actual antigen in the crude antigen preparation (Ag) is indirectly labelled in this way, and that the labelled crude antigen preparation (Ag) is then added to a preincubated reaction mixture which contains the autoantibodies (Ak) to be determined bound via the unspecific binder to a solid or undissolved phase.

7. Assay method according to claim 6, wherein all reaction partners are incubated in a common reaction mixture and the amount of label is determined which is bound to the solid phase after the separation of said solid phase from the liquid phase.

8. Use of crude native autoantigens (Ag) in the form of organ extracts for the preparation of kits for the determination of autoantibodies (Ak) in biological fluids by an immunological assay method according to any of claims 1 to 7.

## Patentansprüche

1. Immunologisches Bestimmungsverfahren für die Bestimmung von Autoantikörpern (Ak) in biologischen Flüssigkeiten, bei dem die biologische Flüssigkeit umgesetzt wird mit
a) einem unspezifischen Binder für Antikörper des zu bestimmenden Antikörpertyps, wobei der genannte Binder in der Reaktionsmischung unlöslich ist oder an eine Festphase oder Mikrofestphase gebunden ist,
b) einem Antigen (Ag) zu den zu bestimmenden Antikörpern (Ak), und
c) einem selektiven Markierungsreagens in Form eines Antikörpers oder eines Antikörperfragments (F(Ak)*), die einen detektierbaren Markierungsanteil aufweisen und spezifisch an das Antigen (Ag) binden,
und bei dem die feste oder ungelöste Phase anschließend von der flüssigen Phase getrennt wird und die Anwesenheit oder Menge des zu bestimmenden Antikörpers (Ak) in der biologischen Flüssigkeit anhand der Menge des markierten Antikörpers oder markierten Antikörperfragments (F(Ak)*) nachgewiesen werden, die indirekt über den unspezifischen Binder an die feste oder ungelöste Phase gebunden sind, und wobei die Anwesenheit oder die Menge der in der biologischen Probe zu bestimmenden Antikörper anschließend durch qualitative Bewertung oder rechnerische Auswertung der erhaltenen Ergebnisse unter Verwendung von Eichkurven bestimmt werden,
dadurch gekennzeichnet, daß zur Bestimmung von humanen Autoantikörpern (Ak), deren Nachweis die Diagnose einer Autoimmunerkrankung ermöglicht, das Antigen (Ag) als eine rohe Autoantigen-Präparation verwendet wird, und wobei, als selektives Markierungsreagens, ein markierter monoklonaler Antikörper oder ein markiertes Fragment eines monoklonalen Antikörpers (F(Ak)*) verwendet werden, die nicht oder nicht wesentlich von dem unspezifischen Binder gebunden werden und die an eine solche Region des Antigens (Ag) binden, die von dem zu bestimmenden Autoantikörper (Ak) nicht erkannt wird und von der Bindung des Antigens (Ag) an den zu bestimmenden Autoantikörper (Ak) nicht beeinflußt wird.

2. Bestimmungsverfahren nach Anspruch 1, bei dem ein humaner oder tierischer Organextrakt als rohe native Autoantigen-Präparation verwendet wird.

3. Bestimmungsverfahren nach Anspruch 1, bei dem der unspezifische Binder ausgewählt ist aus Protein A, Protein G und Anti-Human-Antikörper nicht-humanen Ursprungs.

4. Bestimmungsverfahren nach Anspruch 1, 2 oder 3, bei dem die zu bestimmenden Autoantikörper Autoantikörper gegen Schilddrüsenperoxidase (TPO) sind und bei dem als rohe Antigen-Präparation (Ag) rohe native hTPO in Form eines Extrakts aus zerkleinerten humanen Schilddrüsen verwendet wird.

5. Bestimmungsverfahren nach irgendeinem der Ansprüche 1 bis 4, bei dem der markierte monoklonale Antikörper oder das markierte Fragment eines monoklonalen Antikörpers (F(Ak)*) einen detektierbaren Markierungsanteil enthalten, wie er üblicherweise in immunologischen Bestimmungsverfahren verwendet wird, und der beispielsweise ausgewählt ist aus einem radioaktiven Isotop, einem Enzym, einem Fluoreszenz- oder Chemilumineszenz-Label oder einem Substrat für eine enzymatische Nachweisreaktion.

6. Bestimmungsverfahren nach irgend einem der Ansprüche 1 bis 5, bei dem die rohe native Antigen-Präparation (Ag) in einer vorgeschalteten Stufe mit dem markierten monoklonalen Antikörper oder dem markierten Fragment eines monoklonalen Antikörpers (F(Ak)*) umgesetzt wird und auf diese Weise der Anteil des eigentlichen Antigens in der rohen Antigen-Präparation (Ag) indirekt markiert wird, und daß die markierte rohe Antigen-Präparation (Ag) anschließend einer vorinkubierten Reaktionsmischung zugesetzt wird, die die zu bestimmenden Autoantikörper (Ak) über den unspezifischen Binder an eine feste oder ungelöste Phase gebunden enthält.

7. Bestimmungsverfahren nach Anspruch 6, bei dem alle Reaktionspartner in einer Reaktionsmischung gemeinsam inkubiert werden und die Menge der Markierung, die an die feste Phase gebunden ist, nach der Trennung der genannten festen Phase von der flüssigen Phase bestimmt wird.

8. Verwendung von rohen nativen Autoantigenen (Ag) in Form von Organextrakten für die Herstellung von Kits für die Bestimmung von Autoantikörpern (Ak) in biologischen Flüssigkeiten nach einem immunologischen Bestimmungsverfahren nach einem der Ansprüche 1 bis 7.

## Revendications

1. Procédé de détermination immunologique pour la détermination d'auto-anticorps (Ak) dans des fluides biologiques, dans lequel le fluide biologique est mis à réagir avec:
a) un ligand non spécifique pour des anticorps du type des anticorps à déterminer, ledit ligand étant insoluble dans le mélange de réaction ou lié à une phase solide ou à une phase microsolide,
b) un antigène (Ag) pour les anticorps (Ak) à déterminer, et
c) un réactif de marquage sélectif sous la forme d'un anticorps ou d'un fragment d'un anticorps (F(Ak)*) présentant un marqueur détectable et se liant spécifiquement à l'antigène (Ag),
et dans lequel la phase solide ou non dissoute est ensuite séparée de la phase liquide, et la présence ou la quantité de l'anticorps (Ak) à déterminer dans le fluide biologique est détectée au moyen de la quantité d'anticorps marqué ou de fragment d'anticorps marqué (F(Ak)*) indirectement liée à la phase solide ou non dissoute par l'intermédiaire du ligand non spécifique, et la présence ou la quantité des anticorps à déterminer dans l'échantillon biologique est alors déterminée par estimation qualitative ou par évaluation calculée des résultats obtenus, en recourant à des courbes d'étalonnage,
caractérisé en ce que, en vue de déterminer des auto-anticorps humains (Ak) dont la détection permet le diagnostic d'une maladie auto-immune, l'antigène (Ag) est utilisé sous la forme d'une préparation d'auto-antigène brut, et en ce que, comme réactif de marquage sélectif, on utilise un anticorps monoclonal marqué ou un fragment d'anticorps monoclonal marqué (F(Ak)*), qui ne sont pas liés ou ne sont pas significativement liés par le ligand non spécifique, et qui se lient à une région de l'antigène (Ag) qui n'est pas reconnue par l'auto-anticorps (Ak) à déterminer et qui n'est pas affectée par la liaison de l'antigène (Ag) à l'auto-anticorps (Ak) à déterminer.

2. Procédé de détermination selon la revendication 1, dans lequel on utilise comme préparation d'auto-antigène brut naturel un extrait d'organes humains ou animaux.

3. Procédé de détermination selon la revendication 1, dans lequel le ligand non spécifique est choisi parmi une protéine A, une protéine G et des anticorps anti-humains d'origine non humaine.

4. Procédé de détermination selon les revendications 1, 2 ou 3, dans lequel les auto-anticorps à déterminer sont des auto-anticorps dirigés contre la peroxydase de la thyroïde (TPO), et dans lequel on utilise comme préparation d'antigène brut (Ag) de la hTPO naturelle brute, sous la forme d'un extrait de thyroïdes humaines finement divisées.

5. Procédé de détermination selon l'une quelconque des revendications 1 à 4, dans lequel l'anticorps monoclonal marqué ou le fragment d'anticorps monoclonal marqué (F(Ak)*) portent un marqueur détectable habituellement utilisé dans des procédés de détermination immunologique, par exemple choisi parmi un isotope radioactif, une enzyme, un marqueur fluorescent ou chimiluminescent ou un support pour une réaction de détection enzymatique.

6. Procédé de détermination selon l'une quelconque des revendications 1 à 5, dans lequel la préparation d'antigène (Ag) brut naturel est mise à réagir dans une étape précédente avec l'anticorps monoclonal marqué ou avec le fragment d'anticorps monoclonal marqué (F(Ak)*), et la quantité de l'antigène présent dans la préparation d'antigène brut (Ag) est indirectement marquée de cette manière, et en ce que la préparation d'antigène brut marqué (Ag) est alors ajoutée à un mélange de réaction préalablement incubé, qui contient les auto-anticorps (Ak) à déterminer, liés par l'intermédiaire du ligand non spécifique à une phase solide ou non dissoute.

7. Procédé de détermination selon la revendication 6, dans lequel tous les partenaires de réaction sont incubés dans un mélange de réaction commun, et on détermine la quantité de marqueur qui est liée à la phase solide après la séparation de ladite phase solide de la phase liquide.

8. Utilisation d'auto-antigènes bruts naturels (Ag) sous la forme d'extrait d'organes, pour la préparation de trousses pour la détermination d'auto-anticorps (Ak) dans des fluides biologiques par un procédé de détermination immunologique selon l'une quelconque des revendications 1 à 7.
